# EUROPÄISCHE PATENTSCHRIFT

(11) **EP 3 285 791 B1**
(45) Veröffentlichungstag und Bekanntmachung des Hinweises auf die Patenterteilung: **25.03.2020**
(21) Anmeldenummer: 16738365.2
(22) Anmeldetag: 08.06.2016
(51) Int. Cl.: A61K 36/898, A61K 45/06, A61K 9/00, A61K 31/08, A61K 31/155, A61K 31/728, A61K 31/731, A61K 36/04, A61K 36/09, A61K 36/185, A61K 36/48, A61K 36/68, A61K 36/73, A61P 11/04, A61P 11/14

(54) **ZUSAMMENSETZUNG FÜR DIE BEHANDLUNG DES HALS-/RACHENRAUMS**
COMPOSITION FOR THE TREATMENT OF THE THROAT/PHARYNGEAL CAVITY
COMPOSITION POUR LE TRAITEMENT DE LA CAVITÉ LARYNGOPHARYNGÉE

(30) Priorität: 17.08.2015 DE 102015113543
(43) Veröffentlichungstag der Anmeldung: 28.02.2018
(73) Patentinhaber: Maria Clementine Martin Klosterfrau Vertriebsgesellschaft mbH, 50670 Köln (DE)
(72) Erfinder: GALAN SOÚSA, José, 50670 Köln (DE); VESTWEBER, Anna-Maria, 51399 Burscheid (DE)
(74) Vertreter: von Rohr, Hans Wilhelm
(86) Internationale Anmeldenummer: PCT/EP2016/062948
(87) Internationale Veröffentlichungsnummer: WO 2017/028976

(56) Entgegenhaltungen:
- WO-A1-2016/130830
- WO-A1-2017/028977
- DE-U1-202004 016 646
- DE-U1-202006 008 541
- DE-U1-202008 016 832
- DE-U1-202012 001 752
- DE-U1-202013 000 377
- DE-U1-202013 000 446
- DATABASE GNPD [Online] MINTEL; Oktober 2014 (2014-10), anonymous: "Anginetten", XP002761487, gefunden im www.gnpd.com accession no. 2707871 Database accession no. 2707871
- Anonymous: "Gebrauchsinformation Anginetten Hals-Spray", , 14. Juli 2016 (2016-07-14), Seiten 1-2, XP055300256, Gefunden im Internet: URL:http://www.klosterfrau.de/fileadmin/pr oductdata/anginetten/anginetten_hs/gebrauc hsinformation_anginetten_hals_spray.pdf [gefunden am 2016-09-06]

## Beschreibung

Die vorliegende Erfindung betrifft das medizinische Gebiet der Therapie von entzündlichen Erkrankungen des Mundraums bzw. des Hals- und Rachenraums.

Insbesondere betrifft die vorliegende Erfindung eine Zusammensetzung zur Behandlung von entzündlichen Erkrankungen des Mundraums bzw. des Hals- und Rachenraums sowie deren Verwendung.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf.

Nicht beschränkende Beispiele für derartige entzündliche Erkrankungen des Mundraums sowie des Hals-/Rachenraums sind z. B. Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Auch Entzündungen im Bereich der Mundhöhle, wie z. B. Stomatitis, Gingivitis, Mundschleimhautläsionen oder dergleichen, zählen zu den vorgenannten Erkrankungen des Mund- und Rachenraums.

Für weitergehende diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden.

Der Begriff der entzündlichen Erkrankungen des Mund- und Rachenraums ist somit sehr weit zu verstehen und umfasst sämtliche entzündliche Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im Allgemeinen durch eine topische Therapie und insbesondere in schwerwiegenderen Fällen gegebenenfalls zusätzliche systemische Therapie. So kann in schwereren Fällen eine systemische Verabreichung von Antibiotika vorgenommen werden. In leichteren Fällen kann zur topischen, symptomatischen Behandlung eine alleinige Verabreichung von Antiseptika und entzündungshemmenden Wirkstoffen (z. B. Antiphlogistika, antiinflammatorisch wirkenden Mittel, Lokalantibiotika etc.) vorgesehen sein. Die Wirkstoffe können beispielsweise in Form von Rachenspülungen, Sprays oder Lutschtabletten verabreicht werden.

Als Wirkstoff zur topischen Behandlung von entzündlichen Prozessen bzw. Erkrankungen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quartäre Ammoniumverbindung mit bakterizider und fungizider Wirkung, welche unter anderem in Lutschtabletten zur Anwendung kommt. Nachteilig bei diesem Wirkstoff ist die Tatsache, dass bei hoher Dosierung und übermäßigem Verzehr Magen-Darm-Beschwerden, Atemnot sowie eine vermehrte Methämoglobinbildung, insbesondere bei Kindern, auftreten kann.

Zudem kann als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") verabreicht werden, welches beispielsweise als Spray oder in Form von Spül- bzw. Gurgellösungen appliziert werden kann. Bei längerer Anwendung und starker Dosierung kann es auch hier zu Magen-Darm-Beschwerden und darüber hinaus zu Geschmacksirritationen kommen. Zudem geht die Anwendung insbesondere in Form Gurgellösungen teilweise mit dem Problem von Verfärbungen an den Zähnen einher.

Des Weiteren kommen Wirkstoffe natürlichen Ursprungs zum Einsatz, so z. B. sogenannte Schleimdrogen oder deren Extrakte, wie z. B. Isländisches Moos (*Lichen islandicus*). So beschreibt beispielsweise die WO 2006/111258 A1 ein reizlinderndes Hustenmittel, welches eine Kombination von Isländischem Moos, Malve und einer Zinkverbindung enthält. Derartige Präparate bieten aber nicht immer den gewünschten Therapieerfolg.

Darüber hinaus gehen die im Stand der Technik bekannten Zusammensetzungen zur Behandlung von entzündlichen Erkrankungen des Hals- und Rachenraumes oftmals mit zwar zeitlich begrenzten, dennoch äußerst unangenehmen Veränderungen des Geschmackssinns bzw. mit Geschmacksirritationen einher.

Die DE 20 2013 000 446 U1 betrifft eine Zusammensetzung in fester oder flüssiger, vorzugsweise fester Dosierung zur Behandlung von Heiserkeit oder Halsschmerzen, welche mindestens ein Polysaccharid, mindestens ein Oberflächendesinfektionsmittel und mindestens ein vorzugsweise saures Glykosaminoglykan enthält.

Weiterhin betrifft die DE 20 2004 016 646 U1 eine pharmazeutische Zusammensetzung in Form einer festen Dosierung zum Lutschen, welche in therapeutisch wirksamen Mengen Octenidin als Oberflächendesinfektionsmittel enthält.

Die DE 20 2012 001 752 U1 betrifft eine pharmazeutische Zusammensetzung in Form einer festen Dosierung für die Behandlung von entzündlichen Erkrankungen des Mund- und Rachenraums, wobei die Zusammensetzung in pharmazeutisch wirksamen Mengen mindestens eine Schleimdroge und Polidocanol als Lokalanästhetikum enthält.

Zudem betrifft die DE 20 2008 016 832 U1 eine Zusammensetzung in Form einer flüssigen Dosierung zur Behandlung des Mund- und Hals-/Rachenraums, wobei die Zusammensetzung als Wirkstoff mindestens eine Schleimdroge, Dexpanthenol und Holunder enthält.

Die DE 20 2013 000 377 U1 betrifft wirkstoffhaltige Nanopartikel für die topische Anwendung auf der Mund- und/oder Rachenschleimhaut, wobei die Nanopartikel mindestens ein Träger- und/oder Matrixmaterial einerseits sowie mindestens ein Dispergiermittel andererseits aufweisen und wobei die Nanopartikel mindestens einen topisch applizierbaren ersten Wirkstoff sowie mindestens einen zweiten Wirkstoff in Form eines vorzugsweise sauren Glykosaminoglykans enthalten.

Schließlich betrifft die DE 20 2006 008 541 U1 eine pharmazeutische Zubereitung in Form einer flüssigen Dosierung, welche eine Kombination von mindestens zwei Schleimdrogen enthält, wobei die Kombination Isländisches Moos und mindestens eine weitere Schleimdroge umfasst.

Vor dem Hintergrund des zuvor geschilderten Standes der Technik liegt der vorliegenden Erfindung die Aufgabe zugrunde, eine Zusammensetzung zur prophylaktischen und/oder therapeutischen Behandlung von entzündlichen Erkrankungen des Mund- und/oder Hals-/Rachenraums bereitzustellen, welche die zuvor geschilderten Probleme des Standes der Technik überwindet oder aber zumindest abschwächt.

Insbesondere liegt der vorliegenden Erfindung die Aufgabe zugrunde, eine Zusammensetzung bereitzustellen, welche sich im Rahmen der Behandlung von entzündlichen Erkrankungen des Mund- und Hals-/Rachenraums durch eine hervorragende Wirkeffizienz sowie durch eine langanhaltende Wirkdauer bei gleichzeitig guter Verträglichkeit auszeichnet.

Zur Lösung des zuvor geschilderten Problems schlägt die vorliegende Erfindung - gemäß einem ersten Aspekt der vorliegenden Erfindung - eine Zusammensetzung, insbesondere eine pharmazeutische Zusammensetzung, gemäß den diesbezüglichen unabhängigen Ansprüchen vor; weitere, insbesondere vorteilhafte Ausgestaltungen der erfindungsgemäßen Zusammensetzung sind Gegenstand der diesbezüglichen Unteransprüche.

Zudem betrifft die vorliegende Erfindung - gemäß einem weiteren und zweiten Aspekt der vorliegenden Erfindung - eine Applikations- bzw. Verabreichungsvorrichtung gemäß den diesbezüglich unabhängigen Ansprüchen.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung ist somit - gemäß einem **ersten** Aspekt vorliegenden Erfindung - eine Zusammensetzung in Form einer flüssigen Dosierung zur Verwendung bei der prophylaktischen oder therapeutischen Behandlung von entzündlichen Erkrankungen des Mundraums und/oder des Hals-/Rachenraums,
wobei die Zusammensetzung in Kombination und in jeweils wirksamen Mengen
(a) Eibisch *(Althaea officinalis L.)* in Form eines Flüssigextraktes und/oder Fluidextraktes mit einem Droge/Extrakt-Verhältnis im Bereich von 0,9 : 1 bis 3: 1 und
(b) Polidocanol in einer Menge im Bereich von 0,1 bis 0,5 Gew.-%, bezogen auf die Zusammensetzung,
enthält und
wobei die Zusammensetzung frei von einem Oberflächendesinfektionsmittel ist und/oder wobei die Zusammensetzung kein Oberflächendesinfektionsmittel enthält.

Im Rahmen der vorliegenden Erfindung ist es auf Basis der erfindungsgemäßen Zusammensetzung mit der binären Wirkstoffkombination überraschend gelungen, eine schnell eintretende und äußerst langanhaltende Wirkung bei der Behandlung von entzündlichen Erkrankungen des Mund- und Hals-/Rachenraums zu erzielen. Insbesondere kann mit der erfindungsgemäßen Zusammensetzung die Schmerz- bzw. Entzündungssymptomatik hocheffizient gelindert werden. Darüber hinaus werden Beeinträchtigungen der Phonation und somit der Stimme schnell gelindert und es wird dem unangenehmen Symptom der Mundtrockenheit vorbeugt.

Der Begriff "pharmazeutische Zusammensetzung" bzw. "pharmazeutische Zubereitung", wie er im Rahmen der vorliegenden Erfindung verwendet wird, ist dabei sehr breit zu verstehen und umfasst nicht nur pharmazeutische Präparate bzw. Pharmazeutika und Arzneimittel als solche, sondern auch sogenannte Medizinprodukte, Lebensmittel oder Nahrungsergänzungsmittel.

Bei dem in der erfindungsgemäßen Zusammensetzung enthaltenden Eibisch handelt es sich um eine pflanzenbasierte Schleimdroge bzw. einen pflanzenbasierten Schleimstoff.

Schleimstoffe bzw. Schleime sind üblicherweise Heteropolysaccharide mit Molekulargewichten von ca. 50.000 bis 2.000.000, welche durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Der saure Pflanzenschleim aus der Eibischwurzei enthält L-Rhamnose, D-Galactose, D-Galacturonsäure und D-Glucuronsäure im molaren Verhältnis von ungefähr 3 : 2 : 3 : 3. Der am einfachsten gebaute Teil des Polysaccharids besteht aus sich wiederholenden Undecasaccharid-Untereinheiten.

Der Eibisch *(Althaea officinalis L.)* selbst, welcher zur Gewinnung der darin enthaltenen Schleimstoffe vorzugsweise in Form der Eibischwurzel, -blätter und - blüten eingsetzt wird, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälten Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, dreibis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wässrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirups Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

In der erfindungsgemäßen Zusammensetzung hat es sich als besonders vorteilhaft erwiesen, Schleimdrogen auf Basis von Eibisch einzusetzen, da sie sich in der erfindungsgemäßen flüssigen Darreichungsform - insbesondere auch im Vergleich zu anderen bekannten Schleimdrogen - in Kombination mit dem erfindungsgemäß eingesetzten Polidocanol sowie den übrigen Komponenten durch eine verbesserte Wirksamkeit im Rahmen der Behandlung von entzündlichen Erkrankungen des Hals- und Rachenraums auszeichnen. Die vorteilhaften Eigenschaften werden insbesondere durch die erfindungsgemäßen Ausführungsbeispiele belegt.

Weiterhin enthält die Zusammensetzung nach der vorliegenden Erfindung Polidocanol als Lokalanästhetikum. Als Lokalanästhetika werden im medizinischen Sprachgebrauch im Allgemeinen Anästhetika bezeichnet, welche lediglich eine örtlich begrenzte betäubende Wirkung entfalten. Polidocanol zeigt im Rahmen der Behandlung von entzündlichen Erkrankungen des Hals- und Rachenraums in der erfindungsgemäßen zielgerichteten Wirkstoffkombination im Vergleich zu anderen Lokalanästhetika eine überlegene Wirkung.

Bei Polidocanol handelt es sich gemäß der Definition des Europäischen Arzneibuchs 6.0 um ein Gemisch von Ethern verschiedener Macrogole mit Fettalkoholen, hauptsächlich Laurylalkohol. Synonym wird Polidocanol auch als Hydroxypolyethoxydodecan, Macrogollaurylether oder Lauromacrogol 400 bezeichnet. Aufgrund des lipophilen Dodecylrests und der hydrophilen Etherkette lässt sich Polidocanol gut mit Wasser mischen. Darüber hinaus besitzt Polidocanol oberflächenaktive Eigenschaften. Im Stand der Technik wird Polidocanol im Gebiet der Medizin insbesondere zur Verödung von Hämorrhoiden oder Besenreisern in das betroffene Gewebe injiziert; darüber hinaus kann Polidocanol im Stand der Technik in Hautsalben zur Schmerz- und Juckreizlinderung der äußeren Haut (Lederhaut bzw. Dermis) verwendet werden. Im Rahmen der vorliegenden Erfindung kann überraschenderweise erstmals eine topische Applikation von Polidocanol ausgehend von einem Spray auf der empfindlichen Schleimhaut realisiert werden, insbesondere im Zusammenwirken mit den übrigen Komponenten wird in vollkommen überraschender Weise keinerlei Reizung der Schleimhäute beobachtet. Darüber hinaus besitzt Polidocanol in Kombination mit den übrigen erfindungsgemäßen Komponenten eine hervorragende mucoadhäsive Wirksamkeit, was insbesondere zu einer langanhaltenden Wirksamkeit führt. Für weitergehende Einzelheiten zum Wirkstoff "Polidocanol" kann verwiesen werden auf Römpp, Chemielexikon, 10. Auflage, Georg Thieme Verlag, Stuttgart / New York, Band 5, Seite 3403, Stichwort: "Polidocanol", sowie auf die darin referierte Literatur, deren diesbezüglicher Inhalt hiermit vollumfänglich durch Bezugnahme eingeschlossen ist.

Die vorliegende Erfindung weist zahlreiche weitere Vorteile und Besonderheiten auf, welche sie gegenüber dem Stand der Technik auszeichnen und welche nachfolgend ausführlich geschildet sind:
Mit der erfindungsgemäßen Zusammensetzung, welche erfindungsgemäß als flüssige Dosierung, vorzugsweise in Form eines Sprays, vorliegt, lässt sich in effizienter Weise auch die mit entzündlichen Erkrankungen des Mund- und Hals-/- Rachenraums verbundene Schmerzsymptomatik lindern. Bereits nach einmaliger Anwendung bzw. Applikation werden Halsschmerzen, insbesondere wie sie im Rahmen von grippalen Infekten auftreten, gelindert. Darüber hinaus tritt die schmerzlindernde Wirkung nicht nur sehr schnell ein, sondern hält auch über einen langen Zeitraum an, so dass neben der hohen Wirkeffizienz bzw. Wirksamkeit eine langanhaltende Wirkdauer vorliegt.

Weiterhin können auf Basis der erfindungsgemäßen binären Wirkstoffkombination auch Entzündungen im Mund- und Rachenraum, welche oftmals im Zusammenhang mit grippalen Infekten auftreten, gelindert werden.

Die höhere Wirkeffizienz der erfindungsgemäßen Zusammensetzungen resultiert unter anderem - ohne sich hierbei auf diese Theorie beschränken zu wollen - aus den hervorragenden mucoadhäsiven Eigenschaften der Zusammensetzungen. Insbesondere durch den Einsatz einer Schleimdroge auf Basis von Eibisch wird eine Art Schutzfilm auf der Mund- und Rachenschleimhaut ausgebildet, so dass einerseits eine physikalische Barriere gegenüber Krankheitserregern, wie Bakterien und Viren, gebildet wird und andererseits einem Austrocknen der Schleimhaut vorgebeugt wird. Darüber hinaus wird auf Basis der guten Anheftung der Zusammensetzung an die Schleimhaut die Wirksamkeit der erfindungsgemäßen Zusammensetzung insgesamt verlängert, da die Wirkstoffe, insbesondere auch das Polidocanol, länger am Wirkort verbleiben und somit über einen längeren Zeitraum ihre Wirkung entfalten können. Die mucoadhäsive Wirkung kann zudem durch den zusätzlichen Einsatz von Propylenglykol noch weiterführend verbessert werden.

Darüber hinaus ermöglicht die erfindungsgemäße Zusammensetzung eine äußerst schnelle Regeneration bzw. Wiederherstellung der gestörten Phonation, d. h. der stimmlichen Beeinträchtigungen. Insbesondere Heiserkeit kann in besonders effizienter Weise behandelt werden.

Die erfindungsgemäße Zusammensetzung zeichnet sich gegenüber bekannten Maßnahmen zur Linderung von Halsschmerzen, Heiserkeit und Entzündungssymptomen im Mund- und Rachenraum durch eine schnell eintretende und lang anhaltende Wirkung aus, d. h. die Wirkeffizienz ist insgesamt verbessert. Darüber hinaus kann besonders effizient einem Austrocken der Schleimhäute vorgebeugt bzw. entgegengewirkt werden.

Insgesamt ergänzen sich somit die erfindungsgemäß eingesetzte Schleimdroge auf Basis von Eibisch und das mindestens eine Lokalanästhetikum in synergistischer Weise, da die Wirkung der eingesetzten Komponenten bei kombiniertem Einsatz über die Wirkung der jeweiligen Einzelstoffe bei deren alleinigem Einsatz hinausgeht. Insbesondere in Bezug auf eine Barrierewirkung gegenüber Krankheitserregern, eine Linderung der Schmerz- und Entzündungssymptomatik sowie einer Vorbeugung vor Austrocknung der Schleimhäute ergänzen sich die Inhaltsstoffe synergistisch.

Schließlich zeichnet sich die erfindungsgemäße Zusammensetzung auch durch ihre hervorragende Verträglichkeit aus, da sie zumindest im Wesentlichen frei von etwaigen Nebenwirkungen ist. Aufgrund der guten Verträglichkeit ist die erfindungsgemäße Zusammensetzung somit auch für einen länger andauernden Gebrauch, z. B. bei chronischer Mundtrockenheit, wie sie beispielsweise bei Rauchern auftritt, sowie grundsätzlich auch zur Anwendung bei Kindern geeignet.

Im Zusammenhang mit den zuvor beschriebenen Vorteilen und Besonderheiten der vorliegenden Erfindung wird bereits an dieser Stelle auf die von der Anmelderin durchgeführten und nachfolgend geschilderten Wirksamkeitsstudien verwiesen, welche die vorgenannten Effekte und Eigenschaften in eindrucksvoller Weise belegen. Insbesondere geht aus den durch die Anmelderin durchgeführten Wirksamkeitsstudien hervor, dass die erfindungsgemäße Kombination von Eibisch einerseits und Polidocanol andererseits in flüssiger Darreichungsform gegenüber Zusammensetzungen, welche Kombinationen auf Basis anderer Schleimdrogen, wie z.B. Isländisch Moos oder Malve, und anderer Lokalanästhetika, wie z.B. Benzocain oder Lidocain, enthalten, eine gesteigerte Wirkeffizienz bei der Behandlung von entzündlichen Erkrankungen des Hals- und Rachenraums besitzt.

Die erfindungsgemäße Zusammensetzung kann in vielfältiger Art und Weise ausgestaltet sein, wobei bevorzugte Ausführungsformen im Folgenden näher beschrieben sind.

Im Rahmen der vorliegenden Erfindung kann es üblicherweise vorgesehen sein, dass die Zusammensetzung die Wirk- und/oder Inhaltsstoffe zusammen mit einem unbedenklichen, insbesondere pharmakologisch und/oder physiologisch unbedenklichen Träger und/oder Exzipienten enthält.

Zudem kann es im Rahmen der vorliegenden Erfindung vorgesehen sein, dass der Träger ausgewählt ist aus der Gruppe von Lösungsmitteln, Lösungsvermittlern, Emulgatoren und dergleichen. In diesem Zusammenhang werden besonders gute Ergebnisse erhalten, wenn der Trägerstoff aus der Gruppe von Wasser, Ethanol, Isopropanol, Ethylcarbonat, Ethylacetat, Benzylalkohol, Benzylbenzoat, Propylenglykol, 1,3-Butylglykol sowie deren Kombinationen ausgewählt ist.

Durch den gegebenenfalls vorgesehenen Einsatz eines Trägers bzw. Exzipienten lässt sich die erfindungsgemäße Zusammensetzung weiterführend insbesondere in Bezug auf die Haftungseigenschaften der Wirkstoffe an der Mund- bzw. Rachenschleimhaut modifizieren, was wiederum auch die Wirkdauer der Zusammensetzung verlängert. Eine besonders gute mucoadhäsive Wirkung, d. h. eine gute Anheftung der Zusammensetzung und der darin enthaltenen Wirkstoffe an die Schleimhaut, wird erzielt, wenn der Träger eine Kombination auf Basis von Wasser und Propylenglykol ist. Gleichermaßen kann es vorgesehen sein, dass der Träger eine Kombination von Wasser und Propylenglykol aufweist und/oder hieraus besteht. Darüber hinaus trägt Propylenglykol zu einer verbesserten Konservierung der Zusammensetzung bei. In diesem Zusammenhang ist es zudem vorteilhaft, wenn das gewichtsbezogene Verhältnis von Wasser zu Propylenglykol im Bereich von 100 : 1 bis 1 : 100, insbesondere im Bereich von 50 : 1 bis 1 : 50, vorzugsweise im Bereich von 30 : 1 bis 1 : 30, bevorzugt im Bereich von 15: 1 bis 1: 15, besonders bevorzugt im Bereich von 10: 1 bis 1: 10, liegt. Der Wasseranteil gewährleistet eine komfortable Sprüh- bzw. Applizierbarkeit der erfindungsgemäßen Zusammensetzungen, wohingegen der Anteil an Propylenglykol die Haftung der Zusammensetzung an der Schleimhaut sowie die Konservierung unterstützt.

Was die eingesetzte Menge des Trägers bzw. Exzipienten anbelangt, so kann diese im Allgemeinen in weiten Bereichen variieren: Üblicherweise enthält die Zusammensetzung gemäß der vorliegenden Erfindung den Träger in einer Menge im Bereich von 0,1 bis 90 Gew.-%, insbesondere im Bereich von 0,5 bis 50 Gew.-%, vorzugsweise im Bereich von 1 bis 30 Gew.-%, bevorzugt im Bereich von 5 bis 20 Gew.-%, besonders bevorzugt im Bereich von 7 bis 15 Gew.-%, bezogen auf die Zusammensetzung.

Erfindungsgemäß ist es zudem vorgesehen, dass der Eibisch in Form eines Flüssigextraktes und/oder Fluidextraktes eingesetzt ist. In diesem Zusammenhang ist es im Rahmen der vorliegenden Erfindung bevorzugt, wenn der Eibisch in Form eines wässrigen, alkoholischen oder wässrig-alkoholischen Extrakts eingesetzt ist.

Eine besonders gute Wirksamkeit der erfindungsgemäßen Zusammensetzung wird zudem erzielt, wenn der Eibisch in Form eines Flüssigextraktes mit einem Droge/Extrakt-Verhältnis von mindestens 0,9 : 1 oder mehr eingesetzt ist.

Im Rahmen der vorliegenden Erfindung ist es vorgesehen, dass der Eibisch in Form eines Flüssigextraktes mit einem Droge/Extrakt-Verhältnis im Bereich von 0,9 : 1 bis 3 : 1, noch mehr bevorzugt von etwa 1 : 1, eingesetzt ist.

Das sogenannte Droge/Extrakt-Verhältnis (DEV) charakterisiert das (gewichtsbezogene) Verhältnis von eingesetzter Ausgangsdroge zu erhaltenem Extrakt. Das Droge/Extrakt-Verhältnis (DEV) gibt also an, aus welcher gewichtsbezogenen Menge an eingesetzter Droge (z. B. Eibischwurzel) welche gewichtsbezogene Menge an Extrakt gewonnen ist. Ein Droge/Extrakt-Verhältnis von beispielsweise 2 : 1 bedeutet, dass aus zwei Gewichtsteilen Droge ein Gewichtsteil Extrakt gewonnen wurde. Das Droge/Extrakt-Verhältnis gibt also an, wie viele Gewichtsteile einer Arzneidroge für die Herstellung des gewichtsbezogenen Extraktäquivalents benötigt werden.

Die Verwendung von Extrakten mit definiertem Droge/Extrakt-Verhältnis ist insbesondere vor dem Hintergrund relevant, dass die Qualität des Extraktes einen hohen Einfluss auf die Gesamtqualität der pharmazeutischen Zusammensetzung hat. Vorteilhaft ist es dabei zudem, dass der Extrakt hinsichtlich der Wirkstoffkonzentration gewissermaßen standardisiert ist, so dass auch die finale pharmazeutische Zubereitung qualitativ und quantitativ mit konstant guten bzw. gleichbleibenden Werten bezüglich der Wirk- und Inhaltsstoffe bereitgestellt werden kann.

Was die eingesetzte Menge an Eibisch anbelangt, so kann diese in weiten Bereichen variieren. Besonders gute Ergebnisse werden erfindungsgemäß jedoch erhalten, wenn die Zusammensetzung den Eibisch, vorzugsweise in Form von dessen Extrakt, in einer Menge im Bereich von 0,01 bis 30 Gew.-%, insbesondere im Bereich von 0,1 bis 20 Gew.-%, bevorzugt im Bereich von 1 bis 15 Gew.-%, besonders bevorzugt im Bereich von 8 bis 12,5 Gew.-%, bezogen auf die Zusammensetzung, enthält.

Wie zuvor bereits erwähnt, ist es erfindungsgemäß vorgesehen, dass die Zusammensetzung als Lokalanästhetikum Polidocanol enthält, da dieses in der erfindungsgemäß vorgesehenen Wirkstoffkombination gemeinsam mit Eibisch als Schleimdroge im Vergleich zu anderen Lokalanästhetika bei der Behandlung von entzündlichen Erkrankungen des Hals- und Rachenraums eine überlegene Wirksamkeit zeigt.

Zur Erzielung einer effizienten Schmerzlinderung ist es im Rahmen der vorliegenden Erfindung vorgesehen, dass die Zusammensetzung Polidocanol in einer Menge im Bereich von 0,05 bis 1 Gew.-%, bevorzugt im Bereich von 0,1 bis 0,5 Gew.-%, bezogen auf die Zusammensetzung, enthält.

Weiterhin kann es im Rahmen der vorliegenden Erfindung vorgesehen sein, dass die Zusammensetzung als weitere Komponente Menthol enthält. Bei Menthol handelt es sich um einen monozyklischen Monoterpen-Alkohol, welcher in zahlreichen ätherischen Ölen, insbesondere in Minzöl aus der Pfefferminze (*Mentha* x *piperita*), vorkommt. Menthol ist aufgrund seines frischen Geschmacks einerseits ein Aromastoff, welcher der Zusammensetzung einen angenehmen frischen Geschmack verleiht. Andererseits besitzt Menthol auf der Schleimhaut eine kühlende, juckreizlindernde, schmerzstillende sowie leicht lokalanästhetische und antimikrobielle Wirkung. Durch den Einsatz von Menthol kann die Wirksamkeit der erfindungsgemäßen Zusammensetzungen noch weiter verbessert werden.

In diesem Zusammenhang kann es vorgesehen sein, dass die Zusammensetzung Menthol in einer Menge im Bereich von 0,001 bis 5 Gew.-%, insbesondere im Bereich von 0,01 bis 2 Gew.-%, bevorzugt im Bereich von 0,02 bis 1 Gew.-%, besonders bevorzugt im Bereich von 0,05 bis 0,1 Gew.-%, bezogen auf die Zusammensetzung, enthält.

Darüber hinaus hat sich im Rahmen der vorliegenden Erfindung gezeigt, dass die Stabilität der Zusammensetzung, insbesondere die Stabilität der Inhaltsstoffe, noch weiterführend gesteigert werden kann, wenn die Zusammensetzung mindestens ein Konservierungsmittel enthält. Darüber hinaus kann die Zusammensetzung durch den Einsatz mindestens eines Konservierungsmittels vor mikrobiellem Befall geschützt werden. Als geeignete Konservierungsmittel haben sich Konservierungsmittel aus der Gruppe von Phenolen, aliphatischen oder aromatischen Alkoholen, quartären Ammoniumverbindungen und/oder Carbonsäuren, vorzugsweise Propylenglykolen, Chlorbutanol, Benzylalkohol, Benzalkoniumchlorid, Cetylpyridin, Sorbinsäure und/oder Benzoesäure sowie deren pharmazeutisch verträglichen Salzen, bevorzugt Sorbinsäure und/oder deren pharmazeutisch verträglichen Salzen, erwiesen.

Besonders gute Ergebnisse werden im Rahmen der vorliegenden Erfindung erzielt, wenn das Konservierungsmittel Kaliumsorbat ist. Durch den Einsatz von Kaliumsorbat lassen sich die übrigen Inhaltsstoffe besonders gut stabilisieren. Einerseits bleiben die erfindungsgemäßen Zusammensetzungen bei Einsatz von Kaliumsorbat als Konservierungsmittel besonders lange frei von mikrobiologischem Befall und andererseits ist die Lagerstabilität insgesamt erhöht, d. h. die Zusammensetzungen bleiben auch über einen langen Zeitraum frei von Eintrübungen bzw. Ausflockungen der Inhaltsstoffe und sind somit länger verwendbar.

Was die eingesetzte Menge des Konservierungsmittels anbelangt, so kann diese in weiten Bereichen variieren. Besonders gute Ergebnisse werden im Rahmen der vorliegenden Erfindung erzielt, wenn die Zusammensetzung das Konservierungsmittel in einer Menge im Bereich von 0,001 bis 10 Gew.-%, insbesondere im Bereich von 0,005 bis 5 Gew.-%, vorzugsweise im Bereich 0,01 bis 1 Gew.-%, bevorzugt im Bereich von 0,2 bis 0,3 Gew.-%, bezogen auf die Zusammensetzung, enthält.

Zudem kann es vorgesehen sein, dass die Zusammensetzung mindestens einen weiteren Wirk-, Hilfs-, Additiv- und/oder Inhaltsstoff, insbesondere ausgewählt aus der Gruppe von Verarbeitungshilfsmitteln, Aromastoffen, Geschmacksstoffen, Süßstoffen und Süßungsmitteln, Säuerungsmitteln, Stabilisatoren, Vitaminen, Mineralien und/oder Spurenelementen, enthält.

Darüber hinaus ist es im Rahmen der vorliegenden Erfindung vorgesehen, dass die Zusammensetzung frei von einem Oberflächendesinfektionsmittel, insbesondere frei von Polyhexanid, ist. Gleichermaßen ist es vorgesehen, dass die Zusammensetzung kein Oberflächendesinfektionsmittel, insbesondere kein Polyhexanid, enthält. Im Rahmen der vorliegenden Erfindung hat sich überraschend gezeigt, dass auch ohne den Einsatz eines Oberflächendesinfektionsmittels eine hervorragende Wirksamkeit bei der Behandlung von entzündlichen Erkrankungen des Mund- und Hals-/Rachenraums erzielt werden kann. Auf dieser Basis kann insbesondere das Risiko des Auftretens von Nebenwirkungen, welche im Zusammenhang mit Oberflächendesinfektionsmitteln auftreten, weiter minimiert werden.

In Bezug auf die Dosierung der erfindungsgemäßen Zusammensetzung ist es zudem erfindungsgemäß gemäß einer besonders bevorzugten Ausführungsform vorgesehen, dass die Zusammensetzung in Form einer flüssigen Dosierung, vorzugsweise für die insbesondere topische Sprühapplikation, vorliegt. In diesem Zusammenhang ist es bevorzugt, wenn die Zusammensetzung in einem Applikator bzw. einer Applikationsvorrichtung insbesondere für die Sprühapplikation vorliegt. Auf dieser Basis ist eine komfortable und sichere Anwendung der Zusammensetzung möglich. Darüber hinaus ist die Zusammensetzung auch nach dem ersten Öffnen geschützt vor Keimen, was auch vorteilhaft für deren Haltbarkeit ist.

Die zuvor beschriebene Zusammensetzung nach der Erfindung ist somit insbesondere zur Verwendung bei der prophylaktischen und/oder kurativen Behandlung von Heiserkeit und/oder entzündlichen Erkrankungen des Mundraums und/oder des Hals-/ Rachenraums, insbesondere zur Verwendung bei der topischen Behandlung von Heiserkeit oder Halsschmerzen, geeignet.

Gleichermaßen ist Gegenstand der vorliegenden Erfindung gemäß diesem Erfindungsaspekt die Verwendung einer Zusammensetzung, wie sie zuvor beschrieben wurde, zur Herstellung eines Medikaments zur prophylaktischen und/oder therapeutischen Behandlung, bevorzugt topischen Behandlung, von Heiserkeit und/oder entzündlichen Erkrankungen des Mundraums und/oder des Hals-/ Rachenraums, insbesondere zur topischen Behandlung von Heiserkeit und/oder Halsschmerzen.

Wie zuvor beschrieben wurde, zeichnet sich die erfindungsgemäße Zusammensetzung gegenüber den aus dem Stand der Technik bekannten Maßnahmen durch ihren schnellen Wirkeintritt sowie die lang anhaltende Wirkung im Rahmen der Verwendung zur Behandlung von Heiserkeit und/oder entzündlichen Erkrankungen des Mund- und Rachenraums aus. Insbesondere ermöglicht die erfindungsgemäße Verwendung der zuvor beschriebenen Zusammensetzungen eine rasche Linderung der Schmerz- und Entzündungsymptomatik und gewährleistet gleichzeitig einen effizienten Schutz gegenüber Krankheitserregern, wie Bakterien und Viren.

Für weitergehende Einzelheiten zu der erfindungsgemäßen Verwendung kann - zur Vermeidung unnötiger Wiederholungen - auf obige Ausführungen zu der erfindungsgemäßen Zusammensetzung verwiesen werden, welche in Bezug auf die erfindungsgemäße Verwendung entsprechend gelten.

Weiterer Gegenstand der vorliegenden Erfindung - gemäß einem **zweiten** Aspekt der vorliegenden Erfindung - ist schließlich eine Applikations- und/oder Verabreichungsvorrichtung, insbesondere in Form eines Sprühapplikators, wobei die Applikations- und/oder Verabreichungsvorrichtung eine Zusammensetzung, wie sie zuvor definiert wurde, umfasst.

Für weitergehende Einzelheiten zu der erfindungsgemäßen Verwendung kann - zur Vermeidung unnötiger Wiederholungen - auf obige Ausführungen zu der erfindungsgemäßen Zusammensetzung verwiesen werden, welche in Bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der folgenden Ausführungsbeispiele veranschaulicht, welche die vorliegende Erfindung jedoch keinesfalls beschränken.

### Ausführungsbeispiele:

### A) Herstellung erfindungsgemäßer Zusammensetzung in Form eines Halssprays und von Vergleichszusammensetzungen

Zu Vergleichszwecken werden erfindungsgemäße und nicht erfindungsgemäße Zusammensetzungen jeweils in Form eines Halssprays hergestellt. Als Träger bzw. Exzipient wird Wasser eingesetzt, wobei die übrigen Inhaltsstoffe in dem Träger gelöst vorliegen. Die Herstellung der sprayförmigen Zusammensetzungen erfolgt mit dem Fachmann bekannten Methoden.

Die erfindungsgemäßen Zusammensetzungen A weisen als pharmakologisch relevante Inhaltsstoffe Eibisch-Fluidextrakt mit einem Droge/Extrakt-Verhältnis (DEV) von 1 : 1 sowie Polidocanol als Lokalanästhetikum auf. Der Träger besteht aus einer Kombination von Wasser und Propylenglykol. Die genaue Zusammensetzung ist der nachfolgenden Tabelle zu entnehmen.

**Erfindungsgemäße Zusammensetzung A**

| **Inhaltsstoff** | **Menge (Gew.-%)** |
|---|---|
| Eibisch-Fluidextrakt (DEV 1 : 1) | 0,13 |
| Polidocanol | 0,2 |
| Kaliumsorbat | 0,24 |
| Propylenglykol | 10,00 |
| Wasser | ad 100 |

Darüber hinaus werden erfindungsgemäße Zusammensetzungen B bereitgestellt, welche im Gegensatz zu der erfindungsgemäßen Zusammensetzung A kein Propylenglykol enthalten. Die Rezeptur von Zusammensetzung B ist der nachstehend aufgeführten Tabelle zu entnehmen.

**Erfindungsgemäße Zusammensetzung B**

| **Inhaltsstoff** | **Menge (Gew.-%)** |
|---|---|
| Eibisch-Fluidextrakt (DEV 1 : 1) | 0,13 |
| Polidocanol | 0,2 |
| Kaliumsorbat | 0,24 1 |
| Wasser | ad 100 |

Weiterhin werden die erfindungsgemäßen Zusammensetzungen C und D hergestellt, deren jeweilige Rezepturen sich ebenfalls den nachfolgend aufgeführten Tabellen entnehmen lassen. Die Zusammensetzungen C und D enthalten Polidocanol (Zusammensetzung C) bzw. Eibisch (Zusammensetzung D) im Vergleich zu Zusammensetzung A in reduzierten Mengen.

**Erfindungsgemäße Zusammensetzung C**

| **Inhaltsstoff** | **Menge (Gew.-%)** |
|---|---|
| Eibisch-Fluidextrakt (DEV 1 : 1) | 0,13 |
| Polidocanol | 0,1 |
| Kaliumsorbat | 0,24 |
| Propylenglykol | 10,00 |
| Wasser | ad 100 |

**Erfindungsgemäße Zusammensetzung D**

| **Inhaltsstoff** | **Menge (Gew.-%)** |
|---|---|
| Eibisch-Fluidextrakt (DEV 1 : 1) | 0,065 |
| Polidocanol | 0,2 |
| Kaliumsorbat | 0,24 |
| Propylenglykol | 10,00 |
| Wasser | ad 100 |

Weiterhin werden Zusammensetzungen E bis L bereitgestellt, deren jeweilige Rezeptur sich den nachfolgenden Tabellen entnehmen lässt:

**Zusammensetzung E**

| **Inhaltsstoff** | **Menge (Gew.-%)** |
|---|---|
| Eibisch-Fluidextrakt (DEV 1 : 1) | 0,13 |
| Kaliumsorbat | 0,24 |
| Propylenglykol | 10,00 |
| Wasser | ad 100 |

**Zusammensetzung F**

| **Inhaltsstoff** | **Menge (Gew.-%)** |
|---|---|
| Polidocanol | 0,2 |
| Kaliumsorbat | 0,24 |
| Propylenglykol | 10,00 |
| Wasser | ad 100 |

**Zusammensetzung G**

| **Inhaltsstoff** | **Menge (Gew.-%)** |
|---|---|
| Isländisch Moos (DEV 1 : 1) | 0,13 |
| Polidocanol | 0,2 |
| Kaliumsorbat | 0,24 |
| Propylenglykol | 10,00 |
| Wasser | ad 100 |

**Zusammensetzung H**

| **Inhaltsstoff** | **Menge (Gew.-%)** |
|---|---|
| Malve-Fluidextrakt (DEV 1 : 1) | 0,13 |
| Polidocanol | 0,2 |
| Kaliumsorbat | 0,24 |
| Propylenglykol | 10,00 |
| Wasser | ad 100 |

**Zusammensetzung I**

| **Inhaltsstoff** | **Menge (Gew.-%)** |
|---|---|
| Eibisch-Fluidextrakt (DEV 1 : 1) | 0,13 |
| Benzocain | 0,2 |
| Kaliumsorbat | 0,24 |
| Propylenglykol | 10,00 |
| Wasser | ad 100 |

**Zusammensetzung J**

| **Inhaltsstoff** | **Menge (Gew.-%)** |
|---|---|
| Eibisch-Fluidextrakt (DEV 1 : 1) | 0,13 |
| Lidocain | 0,2 |
| Kaliumsorbat | 0,24 |
| Propylenglykol | 10,00 |
| Wasser | ad 100 |

**Zusammensetzung K**

| **Inhaltsstoff** | **Menge (Gew.-%)** |
|---|---|
| Eibisch-Fluidextrakt (DEV 1 : 1) | 0,13 |
| Polidocanol | 0,2 |
| Propylenglykol | 10,00 |
| Wasser | ad 100 |

**Zusammensetzung L**

| **Inhaltsstoff** | **Menge (Gew.-%)** |
|---|---|
| Eibisch-Fluidextrakt (DEV 1 : 1) | 0,13 |
| Polidocanol | 0,2 |
| Benzalkoniumchlorid | 0,24 |
| Propylenglykol | 10,00 |
| Wasser | ad 100 |

Die Zusammensetzung E enthält als Wirkstoff Eibisch-Fluidextrakt, jedoch kein Polidocanol. Zusammensetzung F hingegen enthält Polidocanol als Wirkstoff, jedoch keinen Eibisch. Bei Zusammensetzung G wird der Eibisch-Fluidextrakt durch die Schleimdroge Isländisch Moos ersetzt. Zusammensetzung H enthält eine Schleimdroge auf Basis von Malve. Zusammensetzung I enthält anstatt des erfindungsgemäß eingesetzten Polidocanols das Lokalanästhetikum Benzocain. Die Zusammensetzung J enthält als Lokalanästhetikum Lidocain. Die Zusammensetzungen K und L sind für die Durchführung von Stabilitätsuntersuchungen vorgesehen, wobei die Zusammensetzung K frei von Konservierungsmitteln ist, d. h. kein Kaliumsorbat enthält. Bei Zusammensetzung L wird das erfindungsgemäß bevorzugte Konservierungsmittel Kaliumsorbat durch Benzalkoniumchlorid ersetzt.

### B) Wirksamkeits- und Anwendungsstudien mit den Halssprays A bis J

Um die Wirksamkeit der erfindungsgemäßen Halssprays zu untersuchen, werden die erfindungsgemäßen Zusammensetzungen A bis D mit den Vergleichszusammensetzungen E bis J bezüglich ihrer Wirkeffizienz miteinander verglichen, indem sie im Rahmen von Anwendungs- und Wirksamkeitsstudien verabreicht werden. Darüber hinaus werden zu Vergleichszwecken im Rahmen der Anwendungs- und Wirksamkeitsstudien Gurgellösungen auf Basis von Salbei und Salz eingesetzt.

Zur Untersuchung der Wirksamkeit der Zusammensetzungen A bis J sowie der Gurgellösungen wurde eine Probandengruppe mit insgesamt 110 Probanden im Alter von 20 bis 70 Jahren, von denen 52 weiblich und 58 männlich waren, herangezogen. Die Probanden litten an mit Heiserkeit und entzündlichen Erkrankungen des Hals- und Rachenraumes einhergehenden grippalen Infekten.

Jeweils 10 Probanden erhielten 3 mal täglich über einen Zeitraum von 3 Tagen jeweils eine der Zusammensetzungen A bis J. 10 weitere Probanden erhielten darüber hinaus über einen Zeitraum von ebenfalls 3 Tagen 3 mal täglich eine Gurgellösung auf Basis von Salzwasser und Salbei. Im Rahmen der Behandlung wurde die jeweils getestete Zusammensetzung bzw. Therapiemaßnahme in Bezug auf eine Linderung der Schmerz- und Entzündungssymptomatik, eine Linderung des Symptoms der Mundtrockenheit, die Nachhaltigkeit der Wirkung sowie eine Verbesserung der Phonation nach dem Schulnotensystem (1 = sehr gut bis 6 = ungenügend) bewertet. Die diesbezüglich erhaltenen Ergebnisse sind der nachstehenden Tabelle 1 zu entnehmen.

**Tabelle 1: Ergebnisse der Anwendungs- und Wirksamkeitsstudien**

| **Zusammensetzung** | **Linderung Schmer zsymptomatik** | **Linderung Entzündungssymptomatik** | **Linderung Mundtrockenheit** | **Nachhaltigkeit der Wirkung** | **Verbesserung der Phonation** |
|---|---|---|---|---|---|
| A | 1,3 | 1,2 | 1,1 | 1,2 | 1,2 |
| B | 1,4 | 1,6 | 1,5 | 1,8 | 1,4 |
| C | 1,6 | 1,3 | 1,4 | 1,7 | 1,4 |
| D | 1,5 | 1,4 | 1,5 | 1,5 | 1,5 |
| E | 4,8 | 4,1 | 3,9 | 4,2 | 4,2 |
| F | 3,7 | 4,2 | 4,2 | 4,3 | 3,9 |
| G | 2,2 | 2,9 | 2,6 | 2,9 | 2,7 |
| H | 2,3 | 3,0 | 2,7 | 2,8 | 2,7 |
| I | 3,3 | 3,2 | 4,3 | 3,9 | 4,0 |
| J | | | | | |
| Gurgellösung | 4,4 | 4,2 | 4,0 | 4,1 | 4,2 |

Mit den erfindungsgemäßen Zusammensetzungen A bis D werden in Bezug auf sämtliche untersuchte Bewertungskriterien hervorragende Ergebnisse erzielt. Die besten Ergebnisse werden insgesamt mit Zusammensetzung A erzielt, welche neben den Hauptwirkstoffen Eibisch und Polidocanol auch Propylenglykol enthält. Bei Verringerung der eingesetzten Menge von Polidocanol bzw. Eibisch (Zusammensetzungen C und D) wird eine leichte Abschwächung der Wirkeffizienz beobachtet. Dennoch weisen auch die Zusammensetzungen C und D immer noch eine hervorragende Wirksamkeit auf. Auf Basis der erfindungsgemäßen Kombination können somit äußerst effizient Hals- und Rachenschmerzen gelindert werden; darüber hinaus kommt es auch in Bezug auf Entzündungssymptome zu einer schnellen Verbesserung. Weiterhin lassen sich mit den erfindungsgemäßen Zusammensetzungen zudem Beeinträchtigungen der Phonation hervorragend behandeln, und auch dem Symptom der Mundtrockenheit kann effektiv vorgebeugt bzw. entgegengestellt werden.

Insgesamt zeichnen sich die erfindungsgemäßen Zusammensetzungen in Bezug auf ihre Wirkung durch eine hervorragende Nachhaltigkeit und Wirkeffizienz aus. Durch den zusätzlichen Einsatz von Propylenglykol kann die Wirksamkeit der erfindungsgemäßen Zusammensetzungen noch weiterführend gesteigert werden, was - ohne sich hierbei auf diese Theorie beschränken zu wollen - vermutlich aus einer Förderung der mucoadhäsiven Wirkung der Zusammensetzung resultiert. Durch Propylenglykol erfolgt eine bessere Haftung der eingesetzten Inhaltsstoffe an der Mund- und Rachenschleimhaut, so dass diese über einen längeren Zeitraum ihre Wirkung am Wirkort entfalten können. Darüber hinaus wird der Schutz gegenüber Krankheitserregern weiterführend verbessert.

Mit den Vergleichszusammensetzungen E bis J sowie der aus dem Stand der Technik bekannten Anwendung von Gurgellösungen können hingegen keine zufriedenstellenden Ergebnisse erzielt werden:
Mit den Zusammensetzungen G und H, welche anstelle von Eibisch als Schleimdroge Isländisch Moos bzw. Malve enthalten, ist die Wirksamkeit der Halssprays gegenüber den erfindungsgemäßen Zusammensetzungen deutlich verringert. Wie zudem aus den Vergleichsbeispielen I und J hervorgeht, wird mit den Lokalanästhetika Benzocain und Lidocain nicht die gleiche hervorragende Wirkung wie mit dem erfindungsgemäß eingesetzten Polidocanol erzielt.

Auch werden mit den Gurgellösungen keine zufriedenstellenden Ergebnisse in Bezug auf die Behandlung von entzündlichen Erkrankungen des Mund- und Rachenraumes erzielt.

Aus den Untersuchungen geht insgesamt hervor, dass die Anwendung der erfindungsgemäßen Zusammensetzungen bei Heiserkeit bzw. entzündlichen Erkrankungen des Mundraums bzw. des Hals- und Rachenraums zu einer signifikanten Linderung der Beschwerden führt; insbesondere tritt eine Linderung der Schmerz- und Entzündungssymptomatik ein. Auch führt die Applikation der erfindungsgemäßen Zusammensetzungen zu einem beschleunigten Rückgang der Beeinträchtigung der Phonation.

Die oben beschriebenen Wirksamkeitsstudien lassen den Schluss zu, dass sich die zielgerichtet ausgewählten und miteinander kombinierten Wirkstoffe Eibisch und Polidocanol - insbesondere in den erfindungsgemäß bevorzugten Mengen - in der flüssigen Darreichungsform synergistisch ergänzen. In der erfindungsgemäßen Wirkstoffkombination zeigt Eibisch bei der Behandlung von entzündlichen Erkrankungen des Hals- und Rachenraums eine verbesserte Wirkeffizienz gegenüber anderen bekannten Schleimdrogen (Isländisch Moos, Malve). Auch Polidocanol zeichnet sich in Kombination mit den übrigen erfindungsgemäß eingesetzten Wirkstoffen gegenüber den bekannten Lokalanästhetika Benzocain und Lidocain durch eine verbesserte Wirksamkeit aus.

### C) Stabilitäts- und Haltbarkeitsuntersuchungen

Weiterhin werden die erfindungsgemäßen Zusammensetzungen A und B, welche Kaliumsorbat und Propylenglykol bzw. Kaliumsorbat enthalten, in Bezug auf ihre Stabilität und Haltbarkeit mit der Zusammensetzung K, welche keinerlei Konservierungsmittel enthält, sowie der Zusammensetzung L, welche Benzalkoniumchlorid als Konservierungsmittel enthält, untersucht. Dazu werden die Zusammensetzungen über einen Zeitraum von 18 Monaten abgedunkelt bei Raumtemperatur, d.h. 22 °C, gelagert. Nach Ablauf von 18 Monaten werden die Zusammensetzungen auf mikrobiellen Befall sowie die Lagerstabilität hin untersucht. Als Indikator für eine verringerte Lagerstabilität wird dabei das Auftreten von Eintrübungen durch ausgefallene Inhaltsstoffe herangezogen. Die diesbezüglich erhaltenen Ergebnisse sind der nachstehenden Tabelle 2 zu entnehmen.

**Tabelle 2: Ergebnisse der Stabilitätsuntersuchungen**

| **Zusammensetzung** | **mikrobieller Befall** | **Eintrübungen** |
|---|---|---|
| A | - | - |
| B | - | + |
| K | ++ | ++ |
| L | - | ++ |

| | | |
|---|---|---|
| -: kein Befall / keine Trübung +: leichter Befall / leichte Trübung, Verwendung noch möglich ++: starker Befall / starke Trübung, Verwendung nicht mehr möglich | | |

Wie die vorstehenden Ausführungen zeigen, werden in Bezug auf die Stabilität und den mikrobiellen Befall die besten Ergebnisse mit der erfindungsgemäßen Zusammensetzung A erzielt, welche sowohl Kaliumsorbat als Konservierungsmittel als auch Propylenglykol enthält. Nach 18 Monaten Lagerzeit wird weder ein mikrobieller Befall noch eine Eintrübung der Zusammensetzungen beobachtet. Auch die erfindungsgemäße Zusammensetzung B, welche kein Propylenglykol enthält, ist nach einer Lagerzeit von 18 Monaten noch verwendbar, allerdings treten bereits leichte Eintrübungen auf, was auf eine geringfügig verringerte Stabilität der Inhaltsstoffe schließen lässt.

Die Zusammensetzung K hingegen weist keine zufriedenstellende Stabilität bzw. Haltbarkeit auf. Bereits nach 18 Monaten ist ein ausgeprägter mikrobieller Befall nachweisbar. Darüber hinaus zeigt die Zusammensetzung starke Eintrübungen. Eine Verwendung der Zusammensetzung ist nach 18 Monaten nicht mehr möglich. Zusammensetzung L, welche Benzalkoniumchlorid als Konservierungsmittel enthält, bleibt zwar frei von mikrobiellem Befall, allerdings weist die Zusammensetzung starke Eintrübungen auf, so dass diese ebenfalls nicht mehr verwendbar ist.

Die obigen Ausführungen zeigen, dass sich durch den Einsatz von Kaliumsorbat einerseits und von Propylenglykol andererseits die Stabilität der erfindungsgemäßen Zusammensetzungen signifikant verbessern lässt. Die Zusammensetzungen können in effizienter Weise vor mikrobiellem Befall geschützt werden. Darüber hinaus wird auch die Lagerstabilität bzw. die Stabilität der Inhaltsstoffe in der Lösung verbessert, so dass die Haltbarkeit insgesamt verlängert wird.

## Patentansprüche

1. Zusammensetzung in Form einer flüssigen Dosierung zur Verwendung bei der prophylaktischen oder therapeutischen Behandlung von entzündlichen Erkrankungen des Mundraums und/oder des Hals-/Rachenraums,
wobei die Zusammensetzung in Kombination und in jeweils wirksamen Mengen
(a) Eibisch *(Althaea officinalis L.)* in Form eines Flüssigextraktes und/oder Fluidextraktes mit einem Droge/Extrakt-Verhältnis im Bereich von 0,9 : 1 bis 3 : 1 und
(b) Polidocanol in einer Menge im Bereich von 0,1 bis 0,5 Gew.-%, bezogen auf die Zusammensetzung,
enthält und
wobei die Zusammensetzung frei von einem Oberflächendesinfektionsmittel ist und/oder wobei die Zusammensetzung kein Oberflächendesinfektionsmittel enthält.

2. Zusammensetzung zur Verwendung nach Anspruch 1, wobei die Zusammensetzung die Wirk- und/oder Inhaltsstoffe zusammen mit einem unbedenklichen, insbesondere pharmakologisch und/oder physiologisch unbedenklichen Träger und/oder Exzipienten enthält.

3. Zusammensetzung zur Verwendung nach Anspruch 2,
wobei der Träger eine Kombination auf Basis von Wasser und Propylenglykol ist und/oder insbesondere wobei der Träger eine Kombination von Wasser und Propylenglykol aufweist und/oder hieraus besteht, insbesondere wobei das gewichtsbezogene Verhältnis von Wasser zu Propylenglykol im Bereich von 100 : 1 bis 1 : 100, insbesondere im Bereich von 50 : 1 bis 1 : 50, vorzugsweise im Bereich von 30 : 1 bis 1 : 30, bevorzugt im Bereich von 15: 1 bis 1: 15, besonders bevorzugt im Bereich von 10 : 1 bis 1 : 10, liegt.

4. Zusammensetzung zur Verwendung nach einem der vorangehenden Ansprüche,
wobei der Eibisch in Form eines wässrigen, alkoholischen oder wässrig-alkoholischen Extrakts eingesetzt ist; und/oder
wobei der Eibisch in Form eines Flüssigextraktes mit einem Droge/Extrakt-Verhältnis von etwa 1 : 1 eingesetzt ist.

5. Zusammensetzung zur Verwendung nach einem der vorangehenden Ansprüche,
wobei die Zusammensetzung den Eibisch, vorzugsweise in Form von dessen Extrakt, in einer Menge im Bereich von 0,01 bis 30 Gew.-%, insbesondere im Bereich von 0,1 bis 20 Gew.-%, bevorzugt im Bereich von 1 bis 15 Gew.-%, besonders bevorzugt im Bereich von 8 bis 12,5 Gew.-%, bezogen auf die Zusammensetzung, enthält.

6. Zusammensetzung zur Verwendung nach einem der vorangehenden Ansprüche,
wobei die Zusammensetzung Menthol enthält, insbesondere in einer Menge im Bereich von 0,001 bis 5 Gew.-%, insbesondere im Bereich von 0,01 bis 2 Gew.-%, bevorzugt im Bereich von 0,02 bis 1 Gew.-%, besonders bevorzugt im Bereich von 0,05 bis 0,1 Gew.-%, bezogen auf die Zusammensetzung.

7. Zusammensetzung zur Verwendung nach einem der vorangehenden Ansprüche, wobei die Zusammensetzung mindestens ein Konservierungsmittel enthält.

8. Zusammensetzung zur Verwendung nach Anspruch 7,
wobei die Zusammensetzung das Konservierungsmittel in einer Menge im Bereich von 0,001 bis 10 Gew.-%, insbesondere im Bereich von 0,005 bis 5 Gew.-%, vorzugsweise im Bereich 0,01 bis 1 Gew.-%, bevorzugt im Bereich von 0,2 bis 0,3 Gew.-%, bezogen auf die Zusammensetzung, enthält.

9. Zusammensetzung zur Verwendung nach einem der vorangehenden Ansprüche, wobei die Zusammensetzung mindestens einen weiteren Wirk-, Hilfs-, Additiv- und/oder Inhaltsstoff, insbesondere ausgewählt aus der Gruppe von Verarbeitungshilfsmitteln, Aromastoffen, Geschmacksstoffen, Süßstoffen und Süßungsmitteln, Säuerungsmitteln, Stabilisatoren, Vitaminen, Mineralien und/oder Spurenelementen.

10. Zusammensetzung zur Verwendung nach einem der vorangehenden Ansprüche, wobei die Zusammensetzung frei von Polyhexanid ist und/oder wobei die Zusammensetzung kein Polyhexanid enthält.

11. Zusammensetzung zur Verwendung nach einem der vorangehenden Ansprüche, wobei die Zusammensetzung in Form einer flüssigen Dosierung, vorzugsweise für die Sprühapplikation, vorliegt.

12. Zusammensetzung zur Verwendung nach einem der vorangehenden Ansprüche, wobei die Zusammensetzung in einem Applikator für die Sprühapplikation vorliegt.

13. Applikations- und/oder Verabreichungsvorrichtung in Form eines Sprühapplikators, wobei die Applikations- und/oder Verabreichungsvorrichtung eine Zusammensetzung, wie in einem der vorangehenden Ansprüche definiert, umfasst.

## Claims

1. A composition in the form of a liquid dose for use in the prophylactic or therapeutic treatment of inflammatory diseases of the mouth and/or the throat/pharyngeal cavity,
wherein the composition contains, in combination and in respectively effective quantities of
(a) marshmallow (*althaea officinalis L.*) in the form of a liquid extract and/or fluid extract with a drug/extract ratio in the range from 0.9:1 to 3:1, and
(b) polidocanol in a quantity in the range from 0.1% to 0.5% by weight in relation to the composition,
and
wherein the composition is free from a surface disinfectant and/or wherein the composition contains no surface disinfectant.

2. The composition for use according to claim 1, wherein the composition contains the active ingredients and/or ingredients together with a harmless, in particular pharmacologically and/or physiologically harmless carrier and/or excipient.

3. The composition for use according to claim 2,
wherein the carrier is a combination based on water and propylene glycol and/or in particular wherein the carrier has and/or consists of a combination of water and propylene glycol, in particular wherein the weight-based ratio of water to propylene glycol is in the range from 100:1 to 1:100, in particular in the range from 50:1 to 1:50, preferably in the range from 30:1 to 1:30, more preferably in the range from 15:1 to 1:15, particularly preferably in the range from 10:1 to 1:10.

4. The composition for use according to any one of the preceding claims,
wherein the marshmallow is used in the form of an aqueous, alcoholic or aqueous-alcoholic extract; and/or
wherein the marshmallow is used in the form of a liquid extract with a drug/extract ratio of about 1:1.

5. The composition for use according to any one of the preceding claims,
wherein the composition contains the marshmallow, preferably in the form of its extract, in a quantity in the range from 0.01% to 30% by weight, in particular in the range from 0.1% to 20% by weight, preferably in the range from 1% to 15% by weight, more preferably in the range from 8% to 12.5% by weight in relation to the composition.

6. The composition for use according to any one of the preceding claims,
wherein the composition contains menthol, particularly in a quantity in the range from 0.001% to 5% by weight, in particular in the range from 0.01% to 2% by weight, preferably in the range from 0.02% to 1% by weight, particularly preferably in the range from 0.05% to 0.1% by weight in relation to the composition.

7. The composition for use according to any one of the preceding claims, wherein the composition contains at least one preservative.

8. The composition for use according to claim 7,
wherein the composition contains the preservative in a quantity in the range from 0.001% to 10% by weight, in particular in the range from 0.005% to 5% by weight, preferably in the range from 0.01% to 1% by weight, more preferably in the range from 0.2% to 0.3% by weight in relation to the composition.

9. The composition for use according to any one of the preceding claims, wherein the composition contains at least one further active ingredient, auxiliary ingredient, additive ingredient and/or ingredient, in particular selected from the group of processing aids, flavouring agents, flavours, sweeteners and sweetening agents, acidifying agents, stabilisers, vitamins, minerals and/or trace elements.

10. The composition for use according to any one of the preceding claims, wherein the composition is free of polyhexanide and/or wherein the composition contains no polyhexanide.

11. The composition for use according to any one of the preceding claims, wherein the composition is in the form of a liquid dose, preferably for spray application.

12. The composition for use according to any one of the preceding claims, wherein the composition is in an applicator for spray application.

13. An application and/or administration device in the form of a spray applicator, wherein the application and/or administration device comprises a composition as defined in any one of the preceding claims.

## Revendications

1. Composition sous la forme d'un dosage liquide destinée à être utilisée dans le traitement prophylactique ou thérapeutique de maladies inflammatoires de la cavité buccale et/ou de la cavité laryngopharyngée,
ladite composition en association et respectivement en des quantités efficaces contenant
(a) de la guimauve (*Althaea officinalis L*.) sous la forme d'un extrait liquide et/ou d'un extrait fluide avec un rapport médicament/extrait compris dans la plage de 0,9 : 1 à 3 : 1 et
(b) du polidocanol en une quantité comprise dans la plage de 0,1 à 0,5 % en poids, rapportée à la composition,
et
ladite composition étant exempte de désinfectant de surface et/ou ladite composition ne contenant aucun désinfectant de surface.

2. Composition destinée à être utilisée selon la revendication 1, ladite composition contenant les principes actifs et/ou les ingrédients conjointement avec un support et/ou un excipient inoffensif, en particulier pharmacologiquement et/ou physiologiquement inoffensif.

3. Composition destinée à être utilisée selon la revendication 2,
le support étant une combinaison à base d'eau et de propylène glycol et/ou, en particulier le support comprenant, et/ou en étant constitué, une combinaison d'eau et de propylèneglycol, en particulier le rapport pondéral eau/propylèneglycol étant compris dans la plage de 100 : 1 à 1 : 100, en particulier dans la plage de 50 : 1 à 1 : 50, de préférence dans la plage de 30 : 1 à 1 : 30, en particulier dans la plage de 15: 1 à 1 : 15, de manière particulièrement préférée dans la plage de 10 : 1 à 1 : 10.

4. Composition destinée à être utilisée selon l'une des revendications précédentes,
la guimauve étant utilisée sous la forme d'un extrait aqueux, alcoolique ou hydroalcoolique ; et/ou
la guimauve étant utilisée sous la forme d'un extrait liquide avec un rapport médicament/extrait d'environ 1 : 1.

5. Composition destinée à être utilisée selon l'une des revendications précédentes,
ladite composition contenant la guimauve, préférentiellement sous la forme de son extrait, en une quantité comprise dans la plage de 0,01 à 30 % en poids, en particulier dans la plage de 0,1 à 20 % en poids, de préférence dans la plage de 1 à 15 % en poids, de manière particulièrement préférée dans la plage de 8 à 12,5 % en poids, rapportée à la composition.

6. Composition destinée à être utilisée selon l'une des revendications précédentes,
ladite composition contenant du menthol, en particulier en une quantité comprise dans la plage de 0,001 à 5 % en poids, en particulier dans la plage de 0,01 à 2 % en poids, de préférence dans la plage de 0,02 à 1 % en poids, de manière particulièrement préférée dans la plage de 0,05 à 0,1 % en poids, rapportée à la composition.

7. Composition destinée à être utilisée selon l'une des revendications précédentes, ladite composition contenant au moins un agent conservateur.

8. Composition destinée à être utilisée selon la revendication 7,
ladite composition contenant l'agent conservateur en une quantité comprise dans la plage de 0,001 à 10 % en poids, en particulier dans la plage de 0,005 à 5 % en poids, préférentiellement dans la plage de 0,01 à 1 % en poids, de préférence dans la plage de 0,2 à 0,3 % en poids, rapportée à la composition.

9. Composition destinée à être utilisée selon l'une des revendications précédentes, ladite composition contenant au moins un autre principe actif, adjuvant, additif et/ou ingrédient, en particulier choisi dans le groupe constitué par les auxiliaires mise en œuvre, les arômes, les agents de sapidité, les édulcorants et les produits édulcorants, les acidifiants, les stabilisants, les vitamines, les minéraux et/ou les oligo-éléments.

10. Composition destinée à être utilisée selon l'une des revendications précédentes, ladite composition étant exempte de polyhexanide et/ou ladite composition ne contenant aucun de polyhexanide.

11. Composition destinée à être utilisée selon l'une des revendications précédentes, ladite composition se présentant sous la forme d'un dosage liquide, de préférence pour une application par pulvérisation.

12. Composition destinée à être utilisée selon l'une des revendications précédentes, ladite composition se présentant dans un applicateur pour une application par pulvérisation.

13. Dispositif d'application et/ou d'administration sous la forme d'un applicateur par pulvérisation, ledit dispositif d'application et/ou d'administration comprenant une composition, telle que définie dans l'une des revendications précédentes.
